# EUROPEAN PATENT APPLICATION

(11) **EP 4 312 022 A1**
(43) Date of publication of application: **31.01.2024**
(21) Application number: 22187116.3
(22) Date of filing: 27.07.2022
(51) Int. Cl.: G01N 27/12, G01N 33/02, G01N 33/12

(54) **KITCHEN UTENSIL**

(71) Applicant: BIC Violex Single Member S.A., 145 69 Anoixi (GR)
(72) Inventor: Antonakis, Ion - Ioannis, 14569 Anoixi (GR)
(74) Representative: Peterreins Schley

(57) **Abstract**

A kitchen utensil for analyzing food is provided. The kitchen utensil comprises a blade comprising a first surface and a second surface, a handle, a biosensor attached to the first surface of the blade and configured to detect molecules associated with spoiled or poisoned food produces and to output a detection signal corresponding to the detection of the molecules, an electronic circuitry configured to receive the detection signal from the biosensor and to analyze the detection signal from the biosensor, an indication system configured to receive driving signals from the electronic circuitry, wherein the driving signals depend on the analysis of the detection signal, and a power supply that is connected to at least the biosensor, the electronic circuitry, and the indication system.

## Description

### TECHNICAL FIELD

The embodiments described herein relate to a kitchen utensil for analyzing food and an associated computer-implemented method for analyzing food using a kitchen utensil.

### BACKGROUND

At the current time, consumers are becoming more aware of the health risks associated with fresh food produce and are actively looking for food monitoring solutions. This is driven by the fact that a larger group of the global population is developing food allergies, with an increase of more than 50 % between 2013 and 2019. However, available food analyzers in the market are often bulky, expensive, and require the sample to be prepared in a specific manner.

In addition, there may be the danger posed by spoiled food, the condition of which may not be apparent on mere inspection. Especially in the case of quickly perishable protein-containing foods, such as meat, fish, or cheese, histamines can form quickly, which can lead to severe illnesses and discomfort, especially in histamine-intolerant persons. Also, dangerous can be scombroid poisoning, often known by the common name of fish poisoning, which can lead to allergic reactions due to improper storage of fish and the resulting histamine formation. Especially in today's world with rising outdoor temperatures, the closed cold chain of perishable foods cannot be guaranteed as well. In view of this, there is a need for uncomplicated and simple ways of testing food during cooking for possible concentrations of harmful molecules that can cause discomfort in humans.

A kitchen knife is a tool, which is usually used in cooking in any case. A growth of almost 10 % is expected in the global kitchen knife market by 2025 due to the increased popularity of cooking shows. Therefore, a device that could integrate the functionality of a food analyzer in a simple to use common household item would be beneficial for the user.

Recent development and research focus on the development of small and cost-effective graphene-based biosensors that can detect multiple biological molecules in fairly quick timescale. Using the change of electrical resistance based on concentration detection of molecules associated with spoiled or poisoned food opens a wide field of applications in food analyzing.

The preceding facts and said developments combined with the need for analyzing food in a convenient manner lead to the situation that a kitchen utensil can be developed for analyzing food.

### SUMMARY

According to a first aspect, a kitchen utensil for analyzing food is provided. The kitchen utensil comprises a blade comprising a first surface and a second surface, a handle, a biosensor attached to the first surface of the blade and configured to detect molecules associated with spoiled or poisoned food produces and to output a detection signal corresponding to the detection of the molecules, an electronic circuitry configured to receive the detection signal from the biosensor and to analyze the detection signal from the biosensor, an indication system configured to receive driving signals from the electronic circuitry, wherein the driving signals depend on the analysis of the detection signal, and a power supply that is connected to at least the biosensor, the electronic circuitry, and the indication system.

According to a second aspect, a computer-implemented method for analyzing food using a kitchen utensil for analyzing food is provided. The computer-implemented method comprises receiving a detection signal corresponding to a first concentration level of molecules associated with spoiled or poisoned food produces from a biosensor of the kitchen utensil, comparing the first concentration level with a second predetermined concentration level, and outputting driving signals allowing an indication system of the kitchen utensil to indicate spoiled or poisoned food when the first concentration level is higher than the second concentration level.

An effect of the technique of the present disclosure is to provide a kitchen utensil for analyzing food that can conveniently integrate an analysis of fresh food produces to detect multiple different harmful biological molecules on the produce. Integrating the process of food analyzing into the kitchen utensil may reduce the risk for the user suffering from ailments or diseases that may be caused by spoiled food. Moreover, no further steps are required for the user other than to use the kitchen utensil for analyzing food of the present disclosure during cooking.

It is also beneficial for the user that the kitchen utensil disclosed herein may be designed to operate without an external power source such as a battery and is thus advantageous in terms of continuous availability of the kitchen utensil, since, for example, charging operations may not be necessary.

Further, the present disclosure allows an exchange of the biosensor performed by the user and may allow the use of different biosensors which may be configured to detect different kinds of molecules associated with spoiled or poisoned food produces. Additionally, the exchangeability of the biosensor may allow an extension of lifetime of the kitchen utensil beyond the lifetime of a single biosensor.

### BRIEF DESCRIPTION OF THE DRAWINGS

- **Figure 1**: schematically illustrates an example of a kitchen utensil for analyzing food according to an embodiment of the present disclosure.
- **Figure 2**: schematically illustrates an example of a cross-section of the biosensor and the blade according to an embodiment of the present disclosure.
- **Figure 3**: schematically illustrates an example of a computer-implemented method for analyzing food using a kitchen utensil for analyzing food.
- **Figure 4**: schematically illustrates an example of a method for using a kitchen utensil for analyzing food.

### DETAILED DESCRIPTION

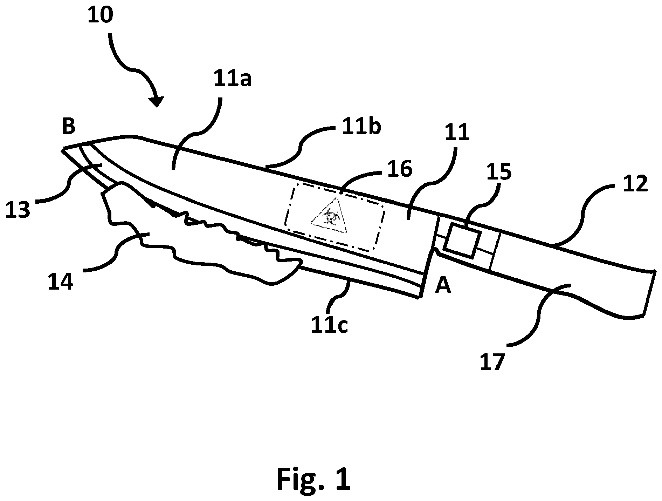

**Fig. 1** schematically illustrates an example of a kitchen utensil 10 for analyzing food according to an embodiment of the present disclosure.

According to the first aspect, the kitchen utensil 10 for analyzing food comprises a blade 11 comprising a first surface 11a and a second surface 11b, a handle 12, a biosensor 13 attached to the first surface 11a of the blade 11 and configured to detect molecules associated with spoiled or poisoned food produces 14 and to output a detection signal corresponding to the detection of the molecules, an electronic circuitry 15 configured to receive the detection signal from the biosensor 13 and to analyze the detection signal from the biosensor 13, an indication system 16 configured to receive driving signals from the electronic circuitry 15, wherein the driving signals depend on the analysis of the detection signal, and a power supply 17 that is connected to at least the biosensor 13, the electronic circuitry 15, and the indication system 16. In an example, the kitchen utensil 10 may have the design of a knife as exemplarily illustrated in **Fig. 1**. In an example, the biosensor 13 may be removable and/or exchangeable. In an example, the molecules associated with spoiled or poisoned food produces 14 may comprise histamine molecules. The histamine molecules may be formed during improper storage of for example quickly perishable protein-containing foods, such as meat, fish, poultry, or cheese. In an example, detecting molecules associated with spoiled or poisoned food produces 14 affects a sensing resistance of the biosensor 13 according to a first concentration level of the molecules associated with spoiled or poisoned food produces 14. For example, the sensing resistance of the biosensor 13 may be direct proportional to the concentration level of the molecules associated with spoiled or poisoned food produces 14, such that in an example, when the first concentration level increases, the sensing resistance of the biosensor 13 may increase. In an example, when the concentration level of the molecules associated with spoiled or poisoned food produces 14 is more than 100 ppm, the resistance may be 30 % higher than when the concentration level is less than 10 ppm. The biosensor 13 may have a detection range of 6.25 ppm - 200 ppm (56.25 µM to 1.8 mM) and may have a low detection limit of 3.41 ppm (30.7 µM).

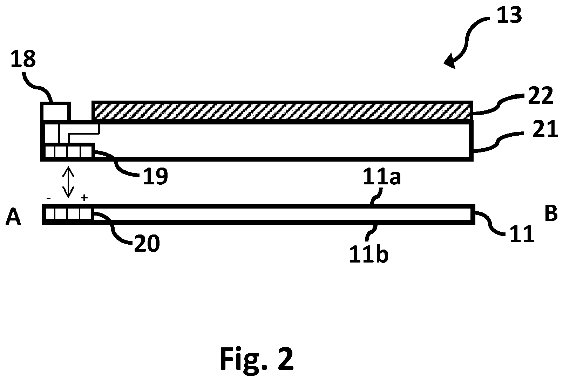

**Fig. 2** schematically illustrates an example of a cross-section of the biosensor 13 and the blade 11 according to an embodiment of the present disclosure.

In an embodiment, the biosensor 13 may comprise a resistor 18, wherein the biosensor 13 may be configured to further output a reference signal according to the resistance value of the resistor 18 and corresponding to a second concentration level of the molecules associated with spoiled or poisoned food produces 14. Fig. 2 shows the resistor 18 in the illustrated exemplary embodiment of the biosensor 13. The resistor 18 may be a passive two-terminal electrical component and may serve as a circuit element. The resistance value of the resistor 18 may be related to the expected sensing resistance of the biosensor in the event molecules associated with spoiled or poisoned food have been detected and hence changing the sensing resistance. In an example, the second concentration level may be a hazardous concentration level for humans. In other words, the resistor 18 serves as an electrical component that lead to a reference signal indicating a second concentration level of molecules associated with spoiled or poisoned food that may have reached a level being hazardous for humans. For example, since the component properties such as type or size of the biosensor 13 affects the sensing resistance, and the reference signal indicates the second concentration level which may be a hazardous concentration level, the resistor may be designed in relation to the component properties of the biosensor 13. For example, when a sensing resistance of the biosensor 13 has a value of 30 kΩ when a first concentration level reaches a hazardous concentration level then the resistance value of the resistor 18 also should be approximately 30 kΩ or at least lead to a reference signal that indicates a second concentration level corresponding to the hazardous concentration level or at least indicates a second concentration level which helps to identify the first concentration level as hazardous. In an example, the resistor 18 may be a SMD resistor, a through-hole resistor, or is formed as a conductive material deposited inside the biosensor 13.

In an embodiment, the biosensor 13 may comprise a plurality of first electrical connectors 19 facing the first surface 1 1a of the blade 11, and the blade 11 may comprise a plurality of second electrical connectors 20 at the first surface 11a of the blade 11 connected to the electronic circuitry 15, wherein the plurality of first electrical connectors 19 and the plurality of second electrical connectors 20 are configured to electrically connect the biosensor 13 to the electronic circuitry 15, when the plurality of first electrical connectors 19 is connected to the plurality of second electrical connectors 20. **Fig. 2** shows an exemplary embodiment of the plurality of first 19 and second 20 electrical connectors in a cross-section. The plurality of first electrical connectors 19 and/or the plurality of second electrical connectors 20 may be formed of a conductive material such as copper or aluminum. In an example, the plurality of first electrical connectors 19 and/or the plurality of second electrical connectors 20 may be formed in the form of pads. In an example, the plurality of first electrical connectors 19 and/or the plurality of second electrical connectors 20 may be formed as a plug-in connection. In an example, plurality of first electrical connectors 19 may be formed as plugs and the plurality of second electrical connectors 20 may be formed as sockets or vice versa.

In an embodiment, the biosensor 13 may comprise a flexible substrate 21 with a sensing area and a second area comprising the plurality of first electrical connectors 19, wherein the second area is on a second surface of the substrate 21 facing the first surface 1 1a of the blade 11 and the sensing area is on a first surface of the substrate 21 opposite the second surface. **Fig. 2** shows an exemplary arrangement of the plurality of first electrical connectors 19 on the second surface of the substrate 21 and the plurality of first electrical connectors 20 on the first surface 11a of the blade 11 facing the plurality of first electrical connectors 19. For example, the plurality of first electrical connectors 19 may be fully embedded or at least partially embedded into the second surface of the substrate 11 such that a protrusion beyond the second surface of the substrate 21 does not equal the full thickness of the plurality of first electrical connectors 19. The resistor 18 may be arranged on the first surface of the substrate 21 and/or may be arranged adjacent to the sensing area.

In an embodiment, the sensing area may comprise a high-resolution conductive electrode array 22, wherein the electrode array 22 may comprise monoclonal antibodies configured to form non-covalent bonds with molecules associated with spoiled or poisoned food produces 14. The non-covalent bonds with molecules associated with spoiled or poisoned food produces 14 may lead to an increase of the sensing resistance of the entire array. The electrode array 22 may comprise a plurality of partial electrode arrays that may be electrically interconnected for example in series or in parallel. The sensing resistance may be a function of the partial sensing resistances of the interconnected partial electrode arrays. In an example, the resulting sensing resistance of the biosensor on either its default state (i.e., no harmful biological molecules) and its maximum concentration state (i.e., the saturation point of the biosensor, or the maximum amount of harmful biological molecules on its surface) and/or in between the default state and the maximum concentration state, may be a function of the sensing array area, array electrode interconnection (parallel or series connections) and monoclonal antibody types bonded to the surface. In an example, at least one electrical connector of the plurality of first electrical connectors 19 and at least one electrical connector of the plurality of second electrical connectors 20 may be configured to transmit the detection signal, and at least one electrical connector of the plurality of first electrical connectors 19 and at least one electrical connector of the plurality of second connectors 20 may be configured to transmit the reference signal. In other words, an electrical connector of the plurality of first electrical connectors 19 may be electrically connected to the resistor 18 which may be arranged on the first surface of the substrate 21 and may be configured to be in contact with the one electrical connector of the plurality of second electrical connectors 20 configured to transmit the reference signal when the biosensor 13 is attached to the first surface 11a of the blade 11, and an electrical connector of the plurality of first electrical connectors 19 may be electrically connected to the sensing area 22 which may be arranged on the first surface of the substrate 21 and may be configured to be in contact with the one electrical connector of the plurality of second electrical connectors 20 configured to transmit the detection signal when the biosensor 13 is attached to the first surface 11a of the blade 11. In an example, as already mentioned, the sensing area may be divided into a plurality of portions, wherein each portion may be configured to detect different kinds of molecules associated with spoiled or poisoned food. For example, each portion may use different monoclonal antibodies, such as for detecting salmonella or molecules leading to scombroid food poisoning such as histamine.

In an embodiment, the biosensor 13 may comprise an adhesive layer on the second surface of the flexible substrate 21 facing the first surface 1 1a of the blade 11 and may be configured to attach the biosensor 13 to the first surface 11a of the blade 11. As mentioned above, the biosensor 13 may be removable and/or exchangeable. For this reason, the biosensor 13 may comprise an adhesive layer such that the biosensor 13 can be attached to the first surface 11a of the blade 11. The adhesive layer may be configured such that the biosensor 13 adheres to the first surface 11a of the blade 11 when the kitchen utensil 10 is used by the user but can be removed from the first surface 11a of the blade 11 by means of a normal traction force that a human can apply without effort. This is to enable the exchangeability of the biosensor 13. For example, after molecules associated with spoiled or poisoned food produces 14 have been detected, replacement of the used biosensor 13 with an unused biosensor may be required. In an example, the adhesive layer may be substituted by another mechanism which may be configured to attach the biosensor 13 to the first surface 11a of the blade 11, such as a plug-in or coupling mechanism or a connection by the means of magnetism. In an example, the first surface 1 1a of the blade 11 may comprise a mounting area providing a mounting location for the biosensor 13, wherein the mounting area may comprise the plurality of second electrical connectors 20 arranged such that the plurality of the second electrical connectors 20 may create an electrical connection with the plurality of first electrical connectors 19 when the biosensor 13 is attached to the first surface 11a of the blade 11. The mounting area may be located along the cutting edge 11c of the blade 11. In an example, the mounting area may provide a counterpart to a plug-in or coupling mechanism in the case the adhesive layer is substituted by a plug-in or coupling mechanism. In the case the biosensor 13 is attached by the means of magnetism, the mounting area may comprise a surface material comprising magnetic materials and/or the blade 11 may be magnetic. In an example, at least one more biosensor 13 may be attached to the first surface 11a and/or the second surface 12a of the blade 11 and the plurality of first electrical connectors 19 and the plurality of second electrical connectors 20 are configured to transmit detection signal and reference signal for each of the at least one more biosensor 13. For example, multiple biosensors 13 may be attached adjacent to the first surface 11a and/or the second surface 11b of the blade 11. Multiple biosensors 13 may allow the detection of different molecules associated with spoiled or poisoned food produces 14 and/or may increase the accuracy of the detection of molecules associated with spoiled or poisoned food produces 14. The second surface 11b of the blade 11 may comprise a plurality of second electrical connectors 20 in addition to the plurality of second electrical connectors 20 at the first surface 1 1a of the blade 11 to allow attachment of at least one biosensor 13 to the second surface 11b of the blade 11. In general, everything that has been described so far for a biosensor 13 attached to the first surface 11a of the blade 11 can also be carried out on the second surface 1 1b of the blade 11 with at least one biosensor in addition to the biosensor on the first surface 11a. Further, there is no limitation in the number of biosensors 13 on both surfaces 1 1a, 11b of the blade 11.

In an embodiment, the electronic circuitry 15 is configured to further receive the reference signal and to output the driving signals allowing the indication system 16 to indicate spoiled or poisoned food when the first concentration level associated with spoiled or poisoned food produces 14 is higher than the second concentration level. When the first concentration level which is related to the detection of molecules associated with spoiled or poisoned food produces 14 is higher than the second concentration level which may serve as a reference concentration level indicating a concentration level being hazardous for humans, the electronic circuitry outputs the driving signals allowing the indication system to indicate spoiled or poisoned food. The indication may be in the form of a visual feedback to the user, such as a warning symbol, a writing, or in the form of colors. In an example, acoustic feedback in the form of a warning sound may be used to warn the user about the detection of molecules associated with spoiled or poisoned food produces 14.

In an embodiment, the electronic circuitry 15 may comprise a computational system configured to compare the current levels/voltage levels of the detection signal and the reference signal, wherein the driving signals are based on the result of the comparison. For example, passing an electrical current through the resistor 18 may cause a voltage drop across the resistor 18 which may result in a reference signal corresponding to the second concentration level. In an example, the current which results when a voltage is applied to the resistor may correspond to the second concentration level. The first concentration level of molecules associated with spoiled or poisoned food produces 14 affects the resistance value of the biosensor 13 which may affect a voltage drop depending on the sensing resistance and therefore results in the detection signal indicating the first concentration level. In the same manner, the current through the biosensor and affected by the concentration level may be used as a detection signal to indicate the first concentration level. In an example, the voltage amplitudes or the current amplitudes of the reference signal and the detection signal may be compared to each other to indicate if the first concentration level exceeds the second concentration level indicating a hazardous concentration level. In the case of the first concentration level exceeds the second concentration level, the computational system generates driving signals which allow the indication system to indicate the danger to the user. In an example, the computational system may comprise a comparator comprising two inputs, wherein the first input may be assigned with the detection signal and a second input may be assigned with the reference signal. For example, the comparator may be configured to compare the voltages or currents of the reference signal and the detection signal, wherein the inputs of the comparator may be analog inputs. The output of the comparator may be a binary signal which may have a high level when the voltage/current amplitude of the detection signal is higher/lower than the voltage/current amplitude of the reference signal and may have low level when the voltage/current amplitude of the detection signal is lower/higher than the voltage/current amplitude of the reference signal, or vice versa in the case of a negation of the output. In an example, the electronic circuitry 15 may comprise display driver electronics configured to convert signals from the computational system into driving signals. The computational system may analyze the detection signal in the view of the reference signal and outputs the result to the display driver electronics. In an example, the display driver electronics may be configured to output driving signals to the indication system 16 allowing the indication system 16 to warn the user, when a hazardous concentration level has been detected. To summarize, the driving signals may allow the indication system 16 to indicate spoiled or poisoned food when the current level/voltage level of the detection signal is lower/higher than the current level/voltage level of the reference signal. In some examples, the driving signals may allow the indication system 16 to indicate spoiled or poisoned food when the current level/voltage level of the detection signal is higher/lower than the current level/the voltage level of the reference signal. In an example, the electronic circuitry 15 may comprise a memory configured to store data associated with the analysis of the detection signal and/or calibration correction parameters. For example, to provide a second concentration level serving as a reference concentration level, reference voltage/current values may be stored in the memory to be compared to the detection signal such that a resistor 18 may not be required for analyzing the detection signal. In an example, there may be stored calibration parameters which may depend on the kind of biosensor 13 used for detection of molecules associated with spoiled or poisoned food produces 14. In other words, different voltage/current values, or in general comparative values, may be stored in the memory for different kinds of biosensors 13 (for example different kinds of antibodies of the electrode arrays). In an example, the electronic circuitry may comprise a processor which may be configured to analyze the detection signal based on the data and/or the calibration correction parameters stored in the memory. For example, the reference signal and/or the detection signal may be analog signals and may get be digitalized by an analog-digital converter in the electronic circuitry 15. The digitalized detection signal may be compared with reference values stored in the memory. In an example, the digitalized reference signals corresponding to the resistor 18 may be compared with the digitalized detection signal from the biosensor 13. In an example, the memory may be an ultra-low power flash memory chip or any kind of a random-access memory (RAM), or a read-only memory (ROM). In the case of an ultra-low power flash memory the power consumption of a complete read and write cycle may be less than 50 µW. In an example, the electronic circuitry may be configured to transmit the result of the analysis of the detection signal to a mobile device. For example, the electronic circuitry may comprise a communication interface configured to communicate with a remote computer system, a mobile device, or any kind of backend or cloud service. The communication interface may be configured to communicate using Bluetooth or Wireless-Fidelity (Wi-Fi). The communication interface may comprise a near-field communication chip. The mobile device or computer system may be configured to visualize the result of the detection signal analysis. In an example, the detection signal may be digitalized by an analog-to-digital converter and may be transmitted to the mobile device. In an example, the biosensor 13 may comprise a (digital) unique identifier to indicate the size and/or type of the biosensor 13. In an example, the unique identifier may be transmitted to the mobile device. For example, the mobile device may be configured to perform an application which may configured to analyze the received digitalized detection signal using the unique identifier.

In an embodiment, the power supply 17 may be embedded in the handle 12 of the kitchen utensil 10. The power supply 17 may be any kind of (rechargeable) battery or may be connected to the fixed mains supply by means of a cable. In an example, the power supply 17 may comprise power electronics 17 which may comprise a transformer and/or a rectifier. The handle 12 may comprise protective insulation against electric shock, such that the user may be protected.

In an example, the power supply 17 may comprise a piezoelectric nanogenerator configured to convert mechanical force exerted by the user while holding the kitchen utensil 10 into electricity. The piezoelectric nanogenerator may be a system constructed from piezoelectric crystals that are capable of converting energy from the mechanical domain to the electrical domain, for example, mechanical force exerted by the user while using the kitchen utensil 10. The piezoelectric crystals may comprise cellulose microfiber and/or flexible liquid metal-tin sulfide. While holding the kitchen utensil 10 the user may exert repeated mechanical stress which can be converted into electrical power by the piezoelectric nanogenerator. In an example, the piezoelectric nanogenerator may generate milli-Volts, such as in the range from 50 mV to 120 mV with a power output of several micro-Watts.

In an embodiment, the power supply 17 may comprise an energy storage which may be configured to store energy generated by the piezoelectric nanogenerator. The energy storage may be a super-capacitor. The piezoelectric nanogenerator in combination with a super-capacitor as an energy storage may be advantageous for the user, since no external energy supply may be required and the kitchen utensil 10 can be used any time without charging a battery or using the mains supply, such that the kitchen utensil 10 can be used everywhere. In an example, the electronic circuitry 15 may comprise electronics configured to set voltage and/or current parameters and regulate the power provided to other components of the electronic circuitry 15, the biosensor 13, and the indication system 16. In an example, the electronic circuitry may be embedded in the handle 12 and/or may be located adjacent to the power supply 17. In an example, at least one electrical connector of the plurality of first electrical connectors 19 and at least one electrical connector of the plurality of second electrical connectors 20 are configured to transmit electrical power from the power supply 17 to the biosensor 13. The electronics may be configured to set voltage and/or current parameters to may allow the power supply to serve as a power supply which may provide an approximately constant current such that a change of the sensing resistance of the biosensor 13 may lead to a voltage drop which may be dependent on the first concentration level of molecules associated with spoiled or poisoned food produces 14. In an example, at least one electrical connector of the plurality of first electrical connectors 19 and at least one electrical connector of the plurality of second electrical connectors 20 transmitting electrical power from the power supply 17 to the biosensor 13 are configured to provide a positive pole, and at least one electrical connector of the plurality of first electrical connectors 19 and at least one electrical connector of the plurality of second electrical connectors 20 are configured to provide a negative pole. For example, the electrode array configured to detect molecules associate with spoiled or poisoned food produces 14 and the resistor 18 may have each electrical connectors providing a positive pole and a negative pole which are separated from each other. As mentioned above, the positive pole and the negative pole may be approximately constant current poles or constant voltage poles. In an example, at least one electrical connector of the plurality of first electrical connectors 19 and at least one electrical connector of the plurality of second electrical connectors 20 transmitting electrical power from the power supply 17 to the biosensor 13 may be configured to provide a positive pole, and at least one electrical connector of the plurality of first electrical connectors 19 and at least one electrical connector of the plurality of second electrical connectors 20 may be configured to electrically connect the biosensor 13 to the blade 11 to provide ground voltage.

**Fig. 1** shows an exemplary location of the indication system 16.

In an embodiment, the indication system 16 may be attached to or embedded in the first surface 11a or the second surface 11b of the blade 11. The indication system 16 may be bonded or mechanically fixed to the first surface 11a or the second surface 11b of the blade 11. In some examples, the indication system may be recessed into the blade such that the outer surface of the indication system 16 facing in the opposite direction of the blade 11 forms a flat surface with the first surface 11a of the blade 11. In an example, the indication system 16 may be protected by a protection layer which may be transparent and may be configured to protect the indication system 16 from external damage that may occur during the use of the kitchen utensil 10, such as dirt or mechanical impact. In an example, the indication system 16 may be embedded in the handle 12 of the kitchen utensil 10. For example, the indication system 16 may be part of the electronic circuitry 16, for example in the case when the indication system 16 may be an acoustic indication system 16 warning the user by any kind of suitable sound. In some examples, the indication system 16 may comprise one of an electronic-ink (e-ink) display, an electrochromic display, a zenithal bistable display (ZBD), a bistable liquid crystal display (LCD), or a cholesteric (or chiral nematic) liquid crystal display (CHLCD). For example, the indication system 16 may comprise at least one single light-emitting diode.

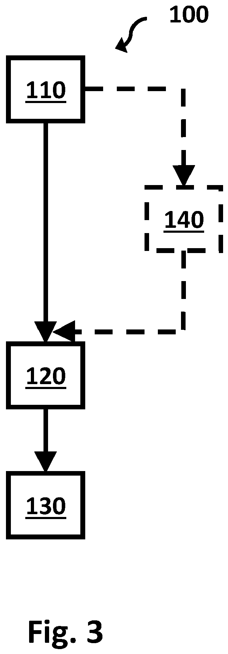

**Fig.** 3 schematically illustrates an example of the computer-implemented method 100 for analyzing food using a kitchen utensil 10 for analyzing food.

According to the second aspect, the computer-implemented method 100 for analyzing food using the kitchen utensil 10 for analyzing food comprises receiving 110 a detection signal corresponding to a first concentration level of molecules associated with spoiled or poisoned food produces 14 from a biosensor 13 of the kitchen utensil 10, comparing 120 the first concentration level with a second predetermined concentration level, and outputting 130 driving signals allowing an indication system 16 of the kitchen utensil 10 to indicate spoiled or poisoned food when the first concentration level is higher than the second concentration level. The computer-implemented method may be performed by a processor included in the electronic circuitry 15. After receiving the detection signal the detection signal will be analyzed for example using the data stored in the memory. In the case the analysis leads to the result that the first concentration level exceeds a second concentration level indicating a hazardous concentration level, the method generates driving signals to be outputted to the indication system 16 to allow the indication system 16 to indicate that the fresh produce 14 is spoiled or poisoned and therefore harmful for humans.

In an embodiment, the method 100 may comprise receiving 140 a reference signal from the biosensor 13, wherein the reference signal may correspond to the second predetermined concentration level, and comparing 120 the first concentration level with the second predetermined concentration level comprises comparing the detection signal with the reference signal. In an example, to get information about the second concentration level the method may comprise receiving 140 the reference signal, such that the detection signal corresponding to the first concentration level may be compared with the reference signal corresponding to the second concentration level indicating a hazardous concentration level.

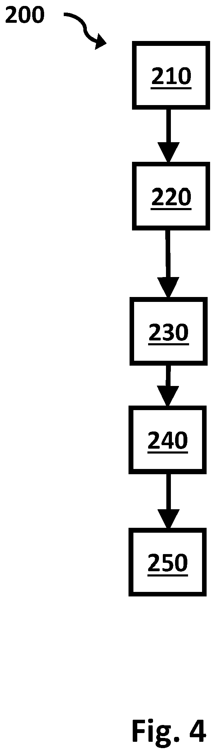

**Fig. 4** schematically illustrates an example of a method for using a kitchen utensil 10 for analyzing food.

Further, a method 200 for using a kitchen utensil for analyzing food is provided. The method 200 may comprise attaching 210 the biosensor to the first surface of the blade 11, applying 220 the smart kitchen utensil 10 for analyzing food to cut a fresh food produce 14 during a cooking process, when the indication system 16 indicates that the fresh food produce 14 is spoiled or poisoned, stopping 230 cutting the fresh food produce 14, and disposing the fresh food produce 14, removing 240 the biosensor 13 from the first surface 11a of the blade 11, and disinfecting 250 the workplace and the kitchen utensil 10. The user may initiate a cooking process of a fresh produce. During the preparation of the recipe the user may be requested to cut a piece of chicken 14 in small strips. While the cutting action is performed, the first surface 11a or the second surface 11b of blade 11 of the kitchen utensil are coming in contact with the contaminated chicken. The action of holding the kitchen utensil 10 and slicing the produce 14 provides power to the device passively through the power supply 17 which may comprise a piezoelectric nanogenerator. The molecules associated with spoiled or poisoned food produces found on the chicken may bind to the antibodies on the surface of the sensing area of the biosensor 13 of the kitchen utensil 10. This in turn may change the sensing resistance of the biosensor 13 indicating potential contamination. As the user may proceed with the cooking (after a predefined amount of time) the indication system 16 may alert the user that there is high potential for contamination. The user can now safely dispose the chicken and may disinfect the workplace. The user may now replace the biosensor 13 with a new biosensor preparing the kitchen utensil 10 for the next time. In the event of no contaminants present on the fresh produce 14, the current biosensor 13 can be reused.

### Embodiments:

1. A kitchen utensil 10 for analyzing food, comprising
   a blade 11 comprising a first surface 11a and a second surface 11b;
   a handle 12;
   a biosensor 13 attached to the first surface 11a of the blade 11 and configured to detect molecules associated with spoiled or poisoned food produces 14 and to output a detection signal corresponding to the detection of the molecules;
   an electronic circuitry 15 configured to receive the detection signal from the biosensor 13 and to analyze the detection signal from the biosensor 13;
   an indication system 16 configured to receive driving signals from the electronic circuitry 15, wherein the driving signals depend on the analysis of the detection signal; and
   a power supply 17 that is connected to at least the biosensor 13, the electronic circuitry 15, and the indication system 16.
2. The kitchen utensil 10 for analyzing food according to embodiment 1, wherein the biosensor 13 is removable.
3. The kitchen utensil 10 for analyzing food according to embodiment 1 or 2, wherein the molecules associated with spoiled or poisoned food produces 14 comprises histamine molecules.
4. The kitchen utensil 10 according to embodiment 1, 2, or 3, wherein the molecules are associated with salmonella.
5. The kitchen utensil 10 for analyzing food according to any one of the preceding embodiments, wherein detecting molecules associated with spoiled or poisoned food produces 14 affects a sensing resistance of the biosensor 13 according to a first concentration level of the molecules associated with spoiled or poisoned food produces 14.
6. The kitchen utensil 10 for analyzing food according to embodiment 5, wherein when the first concentration level increases, the first resistance of the biosensor 13 increases.
7. The kitchen utensil 10 for analyzing food according to embodiment 5 or 6, wherein when the first concentration level increases, the first resistance of the biosensor 13 decreases.
8. The kitchen utensil 10 for analyzing food according to any one of the preceding embodiments, the biosensor 13 comprising a resistor 18, wherein the biosensor 13 is configured to further output a reference signal according to the resistance value of the resistor 18 and corresponding to a second concentration level of the molecules associated with spoiled or poisoned food produces 14.
9. The kitchen utensil 10 for analyzing food according to embodiment 8, wherein the second concentration level is a concentration level being hazardous for humans.
10. The kitchen utensil 10 for analyzing food according to embodiment 8 or 9, wherein the resistor 18 is a SMD resistor, a through-hole resistor, or is formed as a conductive material deposited inside the biosensor 13.
11. The kitchen utensil 10 for analyzing food according to any one of the preceding embodiments, the biosensor 13 comprising a plurality of first electrical connectors 19 facing the first surface 11a of the blade 11, and the blade 11 comprising a plurality of second electrical connectors 20 at the first surface 11a of the blade 11 connected to the electronic circuitry 15, wherein the plurality of first electrical connectors 19 and the plurality of second electrical connectors 20 are configured to electrically connect the biosensor 13 to the electronic circuitry 15, when the plurality of first electrical connectors 19 is connected to the plurality of second electrical connectors 20.
12. The kitchen utensil 10 for analyzing food according to embodiment 11, wherein the biosensor 13 comprises a flexible substrate 21 with a sensing area and a second area comprising the plurality of first electrical connectors 19, wherein the second area is on a second surface of the substrate 21 facing the first surface 11a of the blade 11 and the sensing area is on a first surface of the substrate 21 opposite the second surface.
13. The kitchen utensil 10 for analyzing food according to embodiment 12, wherein the sensing area comprises a high-resolution conductive electrode array 22, wherein the electrode array 22 comprises monoclonal antibodies configured to form non-covalent bonds with molecules associated with spoiled or poisoned food produces 14.
14. The kitchen utensil 10 for analyzing food according to embodiment 11, 12, or 13, wherein at least one electrical connector of the plurality of first electrical connectors 19 and at least one electrical connector of the plurality of second electrical connectors 20 is configured to transmit the detection signal, and at least one electrical connector of the plurality of first electrical connectors 19 and at least one electrical connector of the plurality of second connectors 20 is configured to transmit the reference signal.
15. The kitchen utensil 10 for analyzing food according to embodiment 12, 13 or 14, wherein the sensing area is divided into a plurality of portions, wherein each portion is configured to detect different kinds of molecules associated with spoiled or poisoned food.
16. The kitchen utensil 10 for analyzing food according any one of embodiments 12 to 15, the biosensor 13 comprising an adhesive layer on the second surface of the flexible substrate facing the first surface 11a of the blade 11 and configured to attach the biosensor 13 to the first surface 1 1a of the blade 11.
17. The kitchen utensil 10 for analyzing food according to any one of embodiments 11 to 16, wherein the first surface 11a of the blade 11 comprises a mounting area providing a mounting location for the biosensor 13, wherein the mounting area comprises the plurality of second electrical connectors 20 arranged such that the plurality of the second electrical connectors 20 create an electrical connection with the plurality of first electrical connectors 19 when the biosensor 13 is attached to the first surface 11a of the blade 11.
18. The kitchen utensil 10 for analyzing food according to embodiment 17, wherein the mounting area is located along the cutting edge 11c of the blade 11.
19. The kitchen utensil 10 for analyzing food according to any one of embodiments 11 to 18, wherein at least one more biosensor 13 is attached to the first surface 11a and/or the second surface 12a of the blade 11 and the plurality of first electrical connectors 13 and the plurality of second electrical connectors 20 are configured to transmit detection signal and reference signal for each of the at least one more biosensor 13.
20. The kitchen utensil 10 for analyzing food according to any one of embodiments 5 to 19, wherein the electronic circuitry 15 is configured to further receive the reference signal and to output the driving signals allowing the indication system 16 to indicate spoiled or poisoned food when the first concentration level associated with spoiled or poisoned food produces 14 is higher than the second concentration level.
21. The kitchen utensil 10 for analyzing food according to any one of embodiments 5 to 20, the electronic circuitry 15 comprising a computational system configured to compare the current levels/voltage levels of the detection signal and the reference signal, wherein the driving signals are based on the result of the comparison.
22. The kitchen utensil 10 for analyzing food according to embodiment 21, wherein the computational system comprises a comparator comprising two inputs, wherein a first input is assigned with the detection signal and a second input is assigned with the reference signal.
23. The kitchen utensil 10 for analyzing food according to embodiment 21 or 22, the electronic circuitry 15 comprising display driver electronics configured to convert signals from the computational system into driving signals.
24. The kitchen utensil 10 for analyzing food according to any one of embodiments 8 to 23, wherein the driving signals allow the indication system 16 to indicate spoiled or poisoned food when the current level/voltage level of the detection signal is lower/higher than the current level /the voltage level of the reference signal.
25. The kitchen utensil 10 for analyzing food according to any one of embodiments 8 to 23, wherein the driving signals allow the indication system 16 to indicate spoiled or poisoned food when the current level/ voltage level of the detection signal is higher/lower than the current level /the voltage level of the reference signal.
26. The kitchen utensil 10 for analyzing food according to any one of the preceding embodiments, wherein the electronic circuitry 15 comprises a memory configured to store data associated with the analysis of the detection signal and/or calibration correction parameters.
27. The kitchen utensil 10 for analyzing food according to embodiment 26, wherein the electronic circuitry 15 comprises a processor configured to analyze the detection signal based on the data and/or the calibration correction parameters stored in the memory.
28. The kitchen utensil 10 for analyzing food according to embodiment 26 or 27, wherein the memory is an ultra-low power flash memory chip.
29. The kitchen utensil 10 for analyzing food according to any one of the preceding embodiments, the electronic circuitry 15 comprising a communication interface, wherein the electronic circuitry is configured to transmit the result of the analysis of the detection signal to a mobile device.
30. The kitchen utensil 10 for analyzing food according to any one of the preceding embodiments, wherein the power supply 17 is embedded in the handle 12 of the kitchen utensil 10.
31. The kitchen utensil 10 for analyzing food according to any of the preceding embodiments, wherein the power supply 17 comprises a piezoelectric nanogenerator configured to convert mechanical force exerted by the user while holding the kitchen utensil 10 into electricity.
32. The kitchen utensil 10 for analyzing food according to embodiment 31, wherein the power supply 17 comprises an energy storage configured to store energy generated by the piezoelectric nanogenerator.
33. The kitchen utensil 10 for analyzing food according to embodiment 32, wherein the energy storage is a super-capacitor.
34. The kitchen utensil 10 for analyzing food according to any one of the preceding embodiments, wherein the electronic circuitry 15 comprises electronics configured to set voltage and/or current parameters and regulate the power provided to other components of the electronic circuitry 15, the biosensor 13, and the indication system 16.
35. The kitchen utensil 10 for analyzing food according to any one of the preceding embodiments, wherein the electronic circuitry 15 is embedded in the handle 12.
36. The kitchen utensil 10 for analyzing food according to any one of embodiments 11 to 35, wherein at least one electrical connector of the plurality of first electrical connectors 19 and at least one electrical connector of the plurality of second electrical connectors 20 are configured to transmit electrical power from the power supply 17 to the biosensor 13.
37. The kitchen utensil 10 for analyzing food according to any one of embodiments 11 to 36, wherein at least one electrical connector of the plurality of first electrical connectors 19 and at least one electrical connector of the plurality of second electrical connectors 20 transmitting electrical power from the power supply 17 to the biosensor 13 are configured to provide a positive pole, and at least one electrical connector of the plurality of first electrical connectors 19 and at least one electrical connector of the plurality of second electrical connectors 20 are configured to provide a negative pole.
38. The kitchen utensil 10 for analyzing food according to any one of embodiments 11 to 36, wherein at least one electrical connector of the plurality of first electrical connectors 19 and at least one electrical connector of the plurality of second electrical connectors 20 transmitting electrical power from the power supply 17 to the biosensor 13 are configured to provide a positive pole, and at least one electrical connector of the plurality of first electrical connectors 19 and at least one electrical connector of the plurality of second electrical connectors 20 are configured to electrically connect the biosensor 13 to the blade 11 to provide ground voltage.
39. The kitchen utensil 10 for analyzing food according to any one of the preceding embodiments, wherein the indication system 16 is attached to or embedded in the first surface 1 1a or the second surface 1 1b of the blade 11.
40. The kitchen utensil 10 for analyzing food according to any one of the preceding embodiments, wherein the indication system 16 is embedded in the handle 12 of the kitchen utensil 10.
41. The kitchen utensil 10 for analyzing food according to any one of the preceding embodiments, wherein the indication system 16 comprises one of an electronic-ink (e-ink) display, an electrochromic display, a zenithal bistable display (ZBD), a bistable liquid crystal display (LCD), or a cholesteric (or chiral nematic) liquid crystal display (CHLCD).
42. A computer-implemented method (100) for analyzing food using a kitchen utensil 10 for analyzing food, comprising
   receiving 110 a detection signal corresponding to a first concentration level of molecules associated with spoiled or poisoned food produces 14 from a biosensor 13 of the kitchen utensil 10;
   comparing 120 the first concentration level with a second predetermined concentration level; and
   outputting 130 driving signals allowing an indication system 16 of the kitchen utensil 10 to indicate spoiled or poisoned food when the first concentration level is higher than the second concentration level.
43. The computer-implemented method 100 according to embodiment 42, further comprising
   receiving 140 a reference signal from the biosensor 13, wherein the reference signal corresponds to the second predetermined concentration level, and
   comparing 120 the first concentration level with the second predetermined concentration level comprises comparing the detection signal with the reference signal.
44. A method 200 for using a kitchen utensil for analyzing food, comprising
   attaching 210 the biosensor to the first surface of the blade 11;
   applying 220 the smart kitchen utensil 10 for analyzing food to cut a fresh food produce 14 during a cooking process;
   when the indication system 16 indicates that the fresh food produce 14 is spoiled or poisoned,
   stopping 230 cutting the fresh food produce 14 and disposing the fresh food produce 14, removing 240 the biosensor 13 from the first surface 11a of the blade 11, and disinfecting 250 the workplace and the kitchen utensil 10.

## Claims

1. A kitchen utensil (10) for analyzing food, comprising
a blade (11) comprising a first surface (11a) and a second surface (11b);
a handle (12);
a biosensor (13) attached to the first surface (11a) of the blade (11) and configured to detect molecules associated with spoiled or poisoned food produces (14) and to output a detection signal corresponding to the detection of the molecules;
an electronic circuitry (15) configured to receive the detection signal from the biosensor (13) and to analyze the detection signal from the biosensor (13);
an indication system (16) configured to receive driving signals from the electronic circuitry (15), wherein the driving signals depend on the analysis of the detection signal; and
a power supply (17) that is connected to at least the biosensor (13), the electronic circuitry (15), and the indication system (16).

2. The kitchen utensil (10) for analyzing food according to claim 1, wherein detecting molecules associated with spoiled or poisoned food produces (14) affects a sensing resistance of the biosensor (13) according to a first concentration level of the molecules associated with spoiled or poisoned food produces (14).

3. The kitchen utensil (10) for analyzing food according to any one of the preceding claims, the biosensor (13) comprising a resistor (18), wherein the biosensor (13) is configured to further output a reference signal according to the resistance value of the resistor (18) and corresponding to a second concentration level of the molecules associated with spoiled or poisoned food produces (14) when electrical power from the power supply (17) is provided to the resistor (18).

4. The kitchen utensil (10) for analyzing food according to any one of the preceding claims, the biosensor (13) comprising a plurality of first electrical connectors (19) facing the first surface (11a) of the blade (11), and the blade (11) comprising a plurality of second electrical connectors (20) at the first surface (11a) of the blade (11) connected to the electronic circuitry (15), wherein the plurality of first electrical connectors (19) and the plurality of second electrical connectors (20) are configured to electrically connect the biosensor (13) to the electronic circuitry (15), when the plurality of first electrical connectors (19) is connected to the plurality of second electrical connectors (20).

5. The kitchen utensil (10) for analyzing food according to claim 4, wherein the biosensor (13) comprises a flexible substrate (21) with a sensing area and a second area comprising the plurality of first electrical connectors (19), wherein the second area is on a second surface of the substrate (21) facing the first surface (11a) of the blade (11) and the sensing area is on a first surface of the substrate (21) opposite the second surface.

6. The kitchen utensil (10) for analyzing food according to claim 5, wherein the sensing area comprises a high-resolution conductive electrode array (22), wherein the electrode array (22) comprises monoclonal antibodies configured to form non-covalent bonds with molecules associated with spoiled or poisoned food produces (14).

7. The kitchen utensil (10) for analyzing food according to any one of the preceding claims, wherein the biosensor (13) is removable.

8. The kitchen utensil (10) for analyzing food according to any one of the claims 3 to 7, wherein the electronic circuitry (15) is configured to further receive the reference signal and to output the driving signals allowing the indication system (16) to indicate spoiled or poisoned food when the first concentration level associated with spoiled or poisoned food produces (14) is higher than the second concentration level.

9. The kitchen utensil (10) for analyzing food according to any of one of the claims 3 to 8, the electronic circuitry (15) comprising a computational system configured to compare the current levels/voltage levels of the detection signal and the reference signal, wherein the driving signals are based on the result of the comparison.

10. The kitchen utensil (10) for analyzing food according to any one of the preceding claims, wherein the power supply (17) is embedded in the handle (12) of the kitchen utensil (10).

11. The kitchen utensil (10) for analyzing food according to any of the preceding claims, wherein the power supply (17) comprises a piezoelectric nanogenerator configured to convert mechanical force exerted by the user while holding the kitchen utensil (10) into electricity.

12. The kitchen utensil (10) for analyzing food according to claim 11, wherein the power supply (17) comprises an energy storage configured to store energy generated by the piezoelectric nanogenerator.

13. The kitchen utensil (10) for analyzing food according to any one of the preceding claims, wherein the indication system (16) is attached to or embedded in the first surface (11a) or the second surface (11b) of the blade (11).

14. A computer-implemented method (100) for analyzing food using a kitchen utensil (10) for analyzing food, comprising
receiving (110) a detection signal corresponding to a first concentration level of molecules associated with spoiled or poisoned food produces (14) from a biosensor (13) of the kitchen utensil (10);
comparing (120) the first concentration level with a second predetermined concentration level; and
outputting (130) driving signals allowing an indication system (16) of the kitchen utensil (10) to indicate spoiled or poisoned food when the first concentration level is higher than the second concentration level.

15. The computer-implemented method (100) according to claim 14, further comprising receiving (140) a reference signal from the biosensor (13), wherein the reference signal corresponds to the second predetermined concentration level, and
comparing (120) the first concentration level with the second predetermined concentration level comprises comparing the detection signal with the reference signal.
